(19) Europäisches Patentamt

European Patent Office

Office européen des brevets

(11) **EP 4 667 047 A1**

(12) **EUROPEAN PATENT APPLICATION**

(43) Date of publication:
24.12.2025 Bulletin 2025/52

(21) Application number: 24183385.4

(22) Date of filing: 20.06.2024

(51) International Patent Classification (IPC):
**A61N 1/39** (2006.01)

(52) Cooperative Patent Classification (CPC):
**A61N 1/3937; A61N 1/3981;** A61N 1/3904;
A61N 1/3912

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventor: **ZHOU, Shuai
Eindhoven (NL)**

(74) Representative: **Philips Intellectual Property &
Standards
High Tech Campus 52
5656 AG Eindhoven (NL)**

(54) **CAPACITOR VOLTAGE COMPENSATION IN A DEFIBRILLATOR**

(57) A processing system for controlling the voltage across a capacitor of a defibrillator. The defibrillator is configured to deliver a predetermined energy to a subject upon discharge of the capacitor from an initial voltage. The capacitor has an actual capacitance that is different from a rated capacitance of the capacitor. The processing system is configured to determine the initial voltage from the actual capacitance and a target capacitor energy, being greater than the predetermined energy, and control the defibrillator to charge the capacitor to the initial voltage. The processing system further controls the defibrillator to deliver the predetermined energy to the subject.

FIG. 1

**Description**

FIELD OF THE INVENTION

The present invention relates to the field of defibrillators, and in particular to methods and apparatus for achieving a desired energy output from a defibrillator.

BACKGROUND OF THE INVENTION

Defibrillators are designed to provide an electric shock to a patient's heart for the treatment of life-threatening heart conditions such as cardiac arrest and heart arrythmia. The electric shock provided is often of an intended or predetermined energy, as set by a medical professional. To achieve such an energy output, the defibrillator first stores energy within a capacitor by charging it to an initial voltage, before quickly releasing the stored energy by discharging the capacitor, thereby providing the electric shock to the patient.

The energy stored by a capacitor, when charged to a particular voltage, is directly proportional to the capacitance of the capacitor. Therefore, to accurately provide an electric shock to a patient of an intended energy, the capacitance of the capacitor should preferably be known, e.g., for determining an appropriate voltage to charge the capacitor to prior to providing the electric shock.

Capacitors are manufactured with a nominal or "rated" capacitance. However, it is known that the capacitance of a capacitor may change over time from repeated charging cycles. If this change in capacitance is not accounted for when determining the initial voltage, the electric shock provided to the patient may not be of the intended energy.

There is thus a desire for a system that can account for changes in the capacitance of a capacitor within a defibrillator, such that the electric shock provided to a patient is of an intended energy.

SUMMARY OF THE INVENTION

The invention is defined by the claims.

According to examples in accordance with an aspect of the invention, there is provided a processing system for a defibrillator configured to deliver a predetermined energy to a subject. A capacitor of the defibrillator has an actual capacitance different to a rated capacitance of the capacitor. The processing system is configured to determine an initial voltage responsive to the actual capacitance and a target capacitor energy, being a desired energy stored by the capacitor immediately before delivering the predetermined energy to the subject. The processing system is then configured to control the defibrillator to charge the capacitor to the initial voltage and deliver the predetermined energy to the subject, where the predetermined energy is less than the target capacitor energy.

The present disclosure provides a processing system for determining an initial voltage for a capacitor intended to transfer a predetermined amount of energy from the capacitor to a desired target. More specifically, the processing system is for a defibrillator comprising the capacitor intended to deliver a predetermined energy to a subject (e.g., a patient) for the treatment of a medical condition (e.g., providing electrical energy to a patient's thorax for the treatment of heart arrhythmia). The defibrillator delivers the predetermined energy to the subject upon discharge of the capacitor from the initial voltage to a lower, final voltage.

Prior to delivering an energy output (e.g., the predetermined energy output) the capacitor is charged to a start voltage (i.e., the voltage across the capacitor immediately before the capacitor discharges) to store an amount of initial (electrical) energy within the capacitor. The initial energy stored by the capacitor is proportional to the capacitance of the capacitor multiplied by the square of the voltage across the capacitor (i.e., the start voltage).

The capacitor then discharges from the start voltage to an end voltage (i.e., the voltage across the capacitor immediately after the capacitor stops discharging). By discharging, the capacitor loses (stored) energy and consequently transfers a proportion of the (stored) initial energy to the subject.

In order to provide the predetermined energy to the subject, the capacitor should therefore be charged to an initial voltage that stores a target capacitor energy sufficient for delivery of the predetermined energy. In other words, the target capacitor energy is greater than the predetermined energy.

Determination of the initial voltage requires knowledge of the capacitance of the capacitor. The capacitor has a rated capacitance as given by the manufacturer, i.e., the capacitance measured by the manufacturer when the capacitor was manufactured. However, it has been recognized that an actual capacitance of the capacitor (i.e., the capacitance of the capacitor at a current time) may be different to the rated capacitance due to changes in the capacitance of the capacitor over time (e.g., a gradual decrease in the capacitance from repeated charging cycles of the capacitor).

The proposed processing system is therefore configured to determine the initial voltage responsive to the actual capacitance (rather than the rated capacitance) and the target capacitor energy. The processing system is then configured to control the defibrillator to charge the capacitor to the initial voltage and subsequently deliver the predetermined energy to the subject by discharging the capacitor from the initial voltage.

In some examples, the processing system may be configured to measure the actual capacitance of the capacitor and determine the initial voltage responsive to the measured capacitance.

In particular, the processing system may be configured to measure the actual capacitance by performing a process comprising charging or discharging the capacitor at a constant current and measuring the actual capacitance from the constant current and a rate of change of a voltage across the capacitor.

Specifically, the capacitance of a capacitor can be calculated from the current flowing through the capacitor divided by the rate of change of a voltage across the capacitor. By charging/discharging the capacitor at a constant current, the rate of change of the voltage across the capacitor is similarly constant (i.e., the capacitor charges/discharges linearly). The rate of change of the voltage across the capacitor may thus be calculated from the difference between the voltage across the capacitor at a first time and a second time divided by the time interval between the first time and second time.

In some examples, the target capacitor energy may be associated with the rated capacitance. For example, the target capacitor energy, corresponding with the predetermined energy, may be determined at a time of manufacture of the defibrillator and correspond with a default capacitor energy associated with, i.e., determined responsive to, the rated capacitance.

In some examples, the processing system may be further configured to, prior to charging the capacitor to the initial voltage, determine whether or not the initial voltage breaches a threshold voltage and, in response to the initial voltage breaching the threshold voltage, set the initial voltage to be equal to the threshold voltage.

The threshold voltage may correspond to a maximum (safe) voltage of the capacitor, i.e., a voltage across the capacitor that the capacitor cannot (or should not) be charged above. Accordingly, in response to the target voltage breaching the threshold voltage, the target voltage will be set to the threshold (e.g., maximum) voltage, so that the capacitor is not subsequently charged to a voltage that breaches the threshold voltage.

In some examples, the processing system may be configured to control the defibrillator to deliver the predetermined energy over a target time, corresponding with the time it takes the subject to receive the predetermined energy when discharging the capacitor.

Additionally, the processing system may be configured to determine the target time responsive to the initial voltage, a final voltage, the actual capacitance, an internal resistance of the defibrillator, and a predetermined resistance of the subject, where the final voltage is a desired non-zero voltage across the capacitor after delivering the predetermined energy to the subject.

Furthermore, the processing system may be configured to determine the final voltage responsive to the actual capacitance, the initial voltage, and a total discharge energy, being an expected energy output by the capacitor when delivering the predetermined energy to the subject.

In some examples, the total discharge energy output may be calculated using the formula:

$$W_D = W_S \frac{R_I + R_S}{R_S}$$

where $W_D$ is the total discharge energy, $W_S$ is the predetermined energy output, $R_I$ is the internal resistance of the defibrillator, and $R_S$ is the predetermined resistance of the subject. The above equation comes from treating the internal resistance and the predetermined resistance as two resistors in series.

The predetermined resistance of the subject is related to the patient's contact resistance and their body characteristics. The valid range of patient resistance is 5-325 ohms, for example. In one example, when the user sets the predetermined energy and starts charging, the patient's resistance is not yet known. In this case, a standard resistance (50 ohms) can be used as $R_S$ to calculate the total discharge energy.

In some examples, the processing system may be further configured to display the initial voltage and the actual capacitance on a user interface communicatively coupled to the processing system.

In some examples, the processing system may be further configured to print the initial voltage and the actual capacitance using a printer communicatively coupled to the processing system. It is common for defibrillators to comprise a printer for printing information (e.g., ECG information). This printer may be repurposed for communicating the information about the initial voltage and/or the actual capacitance.

Additionally, or alternatively, the processing system may be configured to determine the actual capacitance of the capacitor no less than once per week. In this way, the actual capacitance is measured on a regular basis.

Further provided is a defibrillator system comprising the processing system according to the above disclosure and the defibrillator configured to deliver the predetermined energy to the subject.

In some examples, the defibrillator system may further comprise a power source switchably coupled to the capacitor of the defibrillator for charging the capacitor to an intended voltage, a voltage measurement system coupled to the capacitor for measuring a voltage across the capacitor, and/or a constant current circuit switchably coupled to the capacitor, configured to permit the capacitor to charge or discharge at a constant current.

The power source may comprise a current source, or a combination of a voltage source (e.g., a battery) and a high voltage transformer, switchably connected (i.e., forms a closed circuit when a switch is closed) in series with the capacitor, able to charge the capacitor up to a maximum (safe) voltage of the capacitor (and any voltage below the maximum voltage). The power source may charge the capacitor (to an intended voltage) for the purposes of measuring the measured capacitance of the capacitor, i.e., through determining the charge/discharge characteristics of the capacitor. Additionally, the power source may charge the capacitor to the initial voltage for the purposes of delivering the predetermined energy output to the subject.

The voltage measurement system may comprise a voltmeter in parallel with the capacitor.

The constant current circuit may comprise a constant current source for driving a constant current through the capacitor. Alternatively, the constant current circuit may comprise an operational amplifier configured to maintain a constant current.

Also provided is a method for charging a capacitor of a defibrillator configured to deliver a predetermined energy to a subject. The capacitor has an actual capacitance different from a rated capacitance of the capacitor. The method comprises determining an initial voltage responsive to the actual capacitance and a target capacitor energy, being a desired energy stored by the capacitor immediately before delivering the predetermined energy to the subject, and charging the capacitor to the initial voltage. Additionally, the predetermined energy is less than the target capacitor energy.

There is also provided a computer program product comprising computer program code means which, when executed on a processing system connected to a defibrillator having a capacitor, cause the processing system to perform all of the steps of the method according to the above disclosure.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a defibrillator system according to an embodiment; and
Fig. 2 shows a flowchart illustrating a proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processing system for a defibrillator configured to deliver a predetermined energy to a subject. The processing system is configured to determine an initial voltage for which to charge a capacitor of the defibrillator to, which delivers the predetermined energy to the subject upon discharge of the capacitor. In other words, the predetermined energy is provided to the subject by discharging the capacitor from the initial voltage, as determined by the processing system, to a lower, final voltage, thereby transferring energy from the capacitor to the subject.

The initial voltage is determined responsive to a capacitance of the capacitor and a desired energy stored by the capacitor immediately before providing the predetermined energy to the subject according to the equation:

$$V = \sqrt{\frac{2W}{C}} \qquad (1)$$

where V is the voltage across the capacitor (e.g., the initial voltage), W is the energy stored by the capacitor and C is the capacitance of the capacitor. Desirably, the capacitor is charged to a voltage which stores a target capacitor energy within the capacitor being greater than the predetermined energy. Advantages of storing an energy within the capacitor greater than the predetermined energy will be described herein. Accordingly, the initial voltage is determined responsive to the target capacitor energy.

The capacitor within the defibrillator has a rated capacitance as assigned and/or measured by a manufacturer. However, it is recognized that the capacitance of a capacitor can change over time. Thus, the initial voltage is also determined responsive to an actual capacitance of the capacitor different to the rated capacitance. Possible techniques of measuring and/or estimating the actual capacitance of the capacitor will be described herein.

Subsequently, the processing system controls the defibrillator to charge the capacitor to the determined initial voltage and then deliver the predetermined energy to the subject upon discharge of the capacitor from the initial voltage to the final voltage.

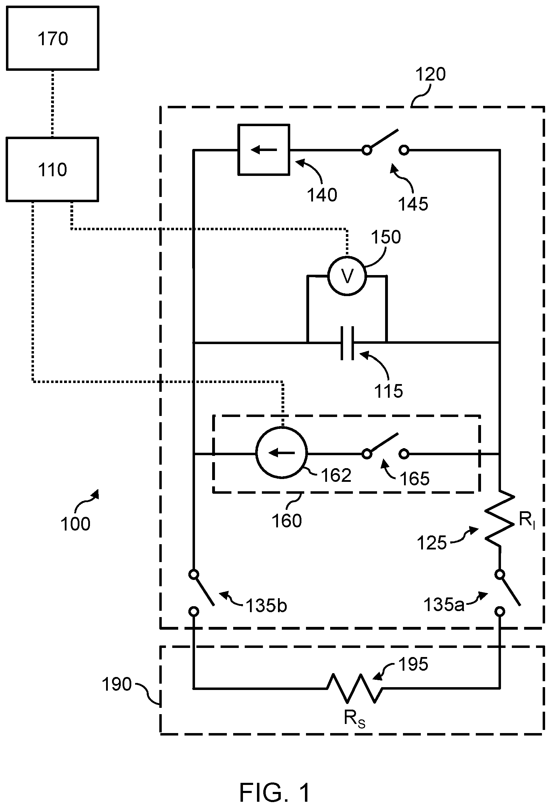

Fig. 1 shows a defibrillator system 100 comprising a processing system 110 and a defibrillator 120 communicatively coupled to the processing system 110. Though the processing system 110 has been depicted in Fig. 1 as being separate from the defibrillator 120, in some embodiments the processing system 110 may form part of, and/or be contained within, the defibrillator 120.

The purpose of the defibrillator 120 is to provide an (electrical) energy output to a subject, e.g., a patient, for the treatment of a medical condition, such as providing an electrical shock to the thorax of a patient suffering from cardiac arrest and/or heart arrythmia. In Fig. 1, the defibrillator 120 has been depicted as a circuit diagram comprising a plurality of electrical components that, in combination, are able to generate/store and subsequently deliver the energy output to the subject. However, those skilled in the art will recognize that the defibrillator 120 may comprise further elements to allow the energy output to be delivered to the subject, e.g., a pair of electrodes for placing in contact with the subject.

For simplicity, a subject 190 has been depicted as being electrically connected to the circuit of the defibrillator 120, characterized by a resistor 195 of a predetermined resistance Rs. Accordingly, the energy output provided to the subject 190 is dissipated within the resistor 195. The predetermined resistance $R_S$ may be an average resistance associated with the human body or may be a resistance associated with a particular part of the human body. Various studies exist in the literature reporting resistances/impedances of the human body. The skilled person will therefore be capable of selecting appropriate values for the predetermined resistance Rs dependent on where on the subject 190 the energy output is being delivered to. For example, the valid range of a subject's resistance is from 5 to 325 ohms. The range may vary greatly depending on the subject. In some instances, a standard resistance equal to 50 ohms can be used as the predetermined resistance Rs. However, it will be appreciated that this may be modified in advance responsive, for instance, to a user input.

The defibrillator comprises a capacitor 115 for storing an amount of (electrical) energy. Prior to delivering the energy output to the subject 190, the capacitor 115 is charged to a start voltage (i.e., charge is provided to the capacitor 115 to create a voltage/potential difference across the capacitor 115 equal to the start voltage) to store energy within the capacitor 115. The energy output is then provided to the subject 190 by discharging the capacitor 115 from the start voltage to an end voltage (i.e., a voltage less than the start voltage), thereby transferring (stored) energy from the capacitor 115 to the subject 190.

The amount of energy provided to the subject 190 is dependent on the total amount of energy lost by the capacitor 115 on discharging from the start voltage to the end voltage, and on the amount of energy transferred (from the capacitor) to elements other than the subject. For example, the defibrillator 120 may be associated with an internal resistance $R_I$ that represents a total combined resistance/impedance of (electrical) elements of the defibrillator 120 that the capacitor discharges through. Consequently, some amount of energy lost by the capacitor 115 on discharging from the start voltage to the end voltage will be dissipated by said elements (e.g., as heat), thereby reducing the amount of energy received by the subject 190. This concept has been represented in Fig. 1 as an internal resistor 125 of internal resistance $R_I$ in series with the capacitor 115 and the subject 190.

The internal resistance $R_I$ may be expected to be in the range 1 to 30 ohms. In some examples, a standard value for $R_I$ may be approximately equal to 12 ohms.

The defibrillator 120 may comprise (or be considered to comprise) one or more switches 135 that, when closed, permit the energy output to be provided to the subject 190. Likewise, after being charged to the start voltage, the capacitor 115 may only discharge (for the purposes of providing the energy output to the subject 190) when the one or more switches 135 is/are closed. More specifically, when closed, the one or more switches 135 form a closed series circuit comprising the capacitor 115, the internal resistor 125 and the resistor 195 (or more generally the subject 190) allowing the capacitor 115 to discharge.

In the example of Fig. 1, the one or more switches 135 comprise two switches 135a and 135b. This is representative of the two paddle/pad/electrode configuration used by defibrillators (such as the defibrillator 120), where each of the switches 135a and 135b is associated with a corresponding paddle of the defibrillator. Closing of the switches 135a and 135b may therefore represent placement on the paddles on appropriate positions on the subject 190, thus forming a closed loop between the subject 190 and the capacitor 115. In other words, the switches 135a and 135b may be closed, or considered to be closed, when the associated paddles are in contact with the subject. Additionally, each of the switches 135a and 135b may also be provided as a physical switch in the associated paddles. The closed series circuit may thus also only be formed when each physical switch is closed.

The one or more switches 135 may be controlled by the processing system 110. In other words, the processing system 110 may be configured to open and/or close the one or more switches 135, e.g., at predefined times and/or when one or more predefined conditions are met, for the purposes of providing the energy output to the subject. In this way, the processing system 110 is able to control the defibrillator 120 to deliver the energy output to the subject 190.

Alternatively, or additionally, the one or more switches 135 may be controlled by a user input. For example, the defibrillator 120 may comprise one or more manual controls (not visible in Fig. 1) e.g., a button on the defibrillator, or one or more buttons on a/the paddle(s) of the defibrillator. When acted on by a user, the one or more manual controls cause the one or more switches 135 to open or close, thereby allowing the user to (manually) control delivery of the energy output to the subject 190.

In one embodiment, a user interacting with the one or more manual controls may trigger a mechanical mechanism to open or close the one or more switches 135. In another embodiment, a user acting on the one or more manual controls may trigger a message/signal to be sent to the processing system 110 that indicates to the processing system 110 to open or close the one or more switches 135.

For the purposes of storing an amount of energy in the capacitor 115 prior to delivering the energy output to the subject, the capacitor 115 may be charged by a power source 140. The power source 140 may be formed as part of (e.g., internal to) the defibrillator, or may be provided externally to the defibrillator, such as a mains power supply to which the defibrillator is configured to electrically connect. The power source 140 may comprise a current source configured to provide electrical charge to the capacitor 115. Alternatively, the power source 140 may comprise a voltage source in combination with a transformer for "stepping up" the voltage provided by the voltage source to a higher voltage (which the capacitor 115 is charged to).

The defibrillator system 100 may comprise a switch 145, e.g., formed as part of the defibrillator 120, that, when closed, permits the power source 140 to charge the capacitor 115. More specifically, when closed, switch 145 forms a closed series circuit comprising the capacitor 115 and the power source 140, allowing current to flow from the power source 140 to the capacitor 115.

Although the internal resistor 125 representing the internal resistance of the defibrillator 120 is not illustrated in Fig. 1 as being part of the described circuit, it will be understood that there will (in practice) be a small or negligible amount of internal resistance present within the described circuit. Preferably, however, the defibrillator will be configured/manufactured to reduce/minimize the internal resistance present when charging the capacitor 115 to improve efficiency.

Switch 145 may be controlled by the processing system 110. In other words, the processing system 110 may be configured to open and/or close switch 145, e.g., at predefined times and/or when one or more predefined conditions are met, for the purposes of charging the capacitor 115 to an intended voltage (e.g., the start voltage).

Alternatively, or additionally, switch 145 may be controlled by a user input. For example, the defibrillator 120 may comprise a manual control (not visible in Fig. 1) e.g., a button, which, when acted on by a user, causes switch 145 to open or close, thereby allowing the user to (manually) control charging of the capacitor 115 by the power source 140.

In one embodiment, a user acting on the manual control may trigger a mechanical mechanism to open or close switch 145. In another embodiment, a user acting on the manual control may trigger a message/signal to be sent to the processing system 110 that indicates to the processing system 110 to open or close switch 145.

Switches 135 and 145 may be inversely coupled such that only one switch may be closed at a time. In this way, the capacitor 115 may not simultaneously be charged by the power source 140 and discharge to provide the energy output to the subject 190. The defibrillator system 100 (e.g., as controlled by the processing system 110) may thus be configured to follow sequential steps of charging the capacitor 115 via the power source 140, and discharging the capacitor through the subject 190.

Note, the above disclosure does not limit switches 135 and 145 from being open at the same time. Particularly, it may be preferable for switches 135 and 145 to be open at the same time when the defibrillator 120 is not in use, and it is therefore unnecessary to charge the capacitor 115 and/or unnecessary to provide an energy output to subject. Additionally, ensuring the switches 135 and 145 are open when the defibrillator is not in use may improve the safety of the defibrillator 120.

The voltage across the capacitor 115 may be determined, recorded and/or monitored using a voltage measurement system 150, such as a voltmeter in parallel with the capacitor 115. The voltage measurement system 150 may be formed as part of the defibrillator 120 (as depicted in Fig. 1) or may simply be a part of the defibrillator system 100 and couple to the capacitor 115 via external connections (e.g., ports) of the defibrillator 120.

The voltage measurement system 150 may also be communicatively coupled to the processing system 110, such that the processing system 110 may receive signals (e.g., electrical signals or wireless signals) from the voltage measurement system 150 indicative of the voltage across the capacitor 115. Responsive to said signals, the processing system 110 may be configured to control switch 145 in order to charge the capacitor 115 to an intended voltage. For example, the processing system 110 may close switch 145 to begin charging the capacitor 115 and subsequently open switch 145 once the voltage across the capacitor 115 reaches the intended voltage.

In practice, a user of the defibrillator 120 may desire to provide an energy output to the subject 190 of a predetermined energy, i.e., a specific amount of energy that the user wishes the subject to receive. Accordingly, the capacitor 115 should be charged to an appropriate start voltage, or initial voltage, which will allow the predetermined energy to be provided to the subject 190 through discharging the capacitor 115. More specifically, an initial voltage should be determined that stores a sufficient amount of energy within the capacitor 115 in order to allow delivery of the predetermined energy to the subject 190.

For example, the predetermined energy may be in the range 1-350 J. More preferably, the predetermined energy may be in the range 120-200 J.

As mentioned previously, the energy lost/transferred by the capacitor 115 on discharging from the start voltage to the end voltage is shared across the internal resistor 125, i.e., the internal resistance of the defibrillator 120, and the resistor 195, i.e., the subject 190. Therefore, in order for the predetermined energy to be provided to the subject 190, the capacitor 115 should be charged to an initial voltage that stores a target capacitor energy greater than the predetermined energy.

By treating the internal resistor 125 and the resistor 195 as being in series (as depicted in Fig. 1) a total discharge energy $W_D$ output by the capacitor 115, required to deliver a predetermined energy Ws to the subject 190, can be determined from the equation:

$$W_D = W_S \frac{R_I + R_S}{R_S} \qquad (2)$$

Therefore, when aiming to provide a predetermined energy Ws to the subject 190, the capacitor 115 should be charged to an initial voltage that stores, at a minimum, a target capacitor energy equal to $W_D$.

Desirably, however, the target capacitor energy will be greater than $W_D$. Accordingly, after delivering the predetermined energy to the subject, there will be a finite amount of energy remaining in the capacitor 115, and therefore a non-zero end voltage, or final voltage, across the capacitor 115.

From equation (1), it is clear that increasing the amount of energy stored by the capacitor 115 will similarly result in (or require) an increased voltage across the capacitor 115. Assuming an exponential behavior, the capacitor 115 will discharge at a rate proportional to the voltage across it (in other words the rate of change of voltage across the capacitor is proportional to the voltage across the capacitor). Therefore, storing a target capacitor energy within the capacitor 115 greater than $W_D$ (and thus charging the capacitor 115 to an initial voltage greater than a minimum required voltage) will cause the capacitor 115 to discharge at a faster rate (at least initially) thereby transferring the predetermined energy to the subject 190 in a shorter amount of time. The subject 190 will therefore receive the predetermined energy as a short, intense "pulse", which may be desirable when the predetermined energy is provided to the subject 190 in the form of an electric shock.

The processing system 110 may be configured to determine the target capacitor energy in response to the predetermined energy. For example, the processing system 110 may have access to a lookup table comprising appropriate target capacitor energies for corresponding predetermined energies. Accordingly, the processing system 110 may determine the target capacitor energy responsive to a requested (e.g., by a user) predetermined energy by referring to the lookup table.

Said lookup table may be stored in a memory (not visible in Fig. 1) formed as part of the defibrillator system 100. The processing system 110 may be coupled to the memory and configured to receive the lookup table from the memory, or receive values from the lookup table by communicating with the memory. Possible examples of memories or memory devices include, but are not limited to, HDDs, SSDs, flash drives, and other forms of magnetic/optical discs and solid-state memory.

The lookup table may be determined at a time of manufacture of the defibrillator 120. Values of the lookup table, i.e., pairs of target capacitor energies and predetermined energies, may be determined responsive to the internal resistance $R_I$ of the defibrillator 120 (e.g., as measured by the manufacturer), a rated capacitance of the capacitor 115, the predetermined resistance Rs, and/or a desired final voltage across the capacitor 115. According to the lookup table, the energy that the capacitor needs to store can be obtained, i.e., by matching a requested predetermined energy with an associated target capacitor energy in the lookup table.

In one embodiment, the processing system 110 may be configured to determine the target capacitor energy by calculating the total discharge energy, e.g., from equation (2), (which represents the minimum energy required to be stored by the capacitor 115 in order to provide the predetermined energy to the subject 190) and applying an amplification factor to the total discharge energy. Put another way, the target capacitor energy may be calculated according to the equation:

$$W_T = W_D * \mu \qquad (3)$$

where $W_T$ is the target capacitor energy and $\mu$ is the amplification factor, being a number greater than 1. Using equation (3), the target capacitor energy is thus calculated to be greater than the total discharge energy.

The amplification factor may preferably be in the range 1.01-1.3 and may be predetermined (e.g., by a manufacturer) or defined (or predefined) by a user. In one practical application, the amplification factor may be equal to 1.07. Accordingly, predetermined energies equal to 50 J, 150 J and 200 J (e.g., as requested by a user) may correspond to target capacitor energies approximately equal to 66.3 J, 199 J and 265.4 J respectively, e.g., when using standard (previously disclosed) values for $R_l$ and Rs.

The processing system 110 may receive a value for the predetermined energy via a user interface 170 formed as part of the defibrillator system 170. The processing system 110 may be communicatively coupled to the user interface 170 and be configured to receive signals from the user interface 170 indicative of the value of the predetermined energy. More specifically, a user may be able to input the value of the predetermined energy into the user interface 170 (e.g., via a keyboard or numerical control) which is then transmitted to the processing system 110. The energy stored in the capacitor prior to delivering the predetermined energy to the subject may therefore correspond to or be defined by a user input.

Though the user interface 170 has been depicted in Fig. 1 as being separate from the defibrillator 120, in some embodiments the user interface 170 may be formed as part of the defibrillator 120.

Prior to delivering the predetermined energy to the subject 190, the capacitor 115 is charged to an initial voltage that stores the target capacitor energy within the capacitor 115. The initial voltage required to store the target capacitor energy is given by equation (1), and is thus dependent on both the target capacitor energy and the capacitance of the capacitor 115.

The capacitor 115 has a rated capacitance corresponding to an intended and/or measured capacitance of the capacitor 115 at the time of manufacture. When the capacitor 115 is installed within the defibrillator 120, it can be assumed that the capacitance of the capacitor 115 is substantially similar to (i.e., within an acceptance range of) the rated capacitance. However, it is known that the capacitance of a capacitor may change over time from repeated charging cycles, e.g., that cause (gradual) degradation of the capacitor plates and/or capacitor dielectric resulting in a (permanent) change to the capacitance of the capacitor.

Therefore, in order to determine an accurate value of the initial voltage required to store the target capacitor energy within the capacitor 115, the initial voltage may be determined responsive to an actual (current) capacitance of the capacitor 115, as opposed to the rated capacitance. More particularly, the processing system is configured to determine the initial voltage responsive to the actual capacitance and the target capacitor energy.

The processing system 110 may be configured to estimate the actual capacitance of the capacitor 115, when calculating the initial voltage from equation (1), using an expected rate of "decay" of the capacitance of the capacitor 115. For example, an estimate of the actual capacitance may be calculated from parameters such as a time since the capacitor 115 was manufactured and/or the number of times the capacitor has been charged and/or discharged. Specifically, said factors may be used to estimate an expected deviation of the capacitance of the capacitor 115 from the rated capacitance, thus providing an estimate of the actual capacitance.

Alternatively, the processing system 110 may be configured to measure the actual capacitance (or more specifically measure an estimate of the actual capacitance) of the capacitor 115. The processing system 110 may then determine the initial voltage responsive to the measured capacitance.

It has been recognized that the capacitance of a capacitor can be determined from measuring the charge/-discharge characteristics of the capacitor. In particular, a current I through a capacitor is given by the capacitance of the capacitor multiplied by a rate of change of voltage across the capacitor with respect to time dV/dt. The current passing through the capacitor and the voltage across the capacitor may be directly measured (e.g., from using an ammeter in series with the capacitor and a voltmeter in parallel with the capacitor) allowing the capacitance of the capacitor to be determined from the equation:

$$C = I \left( \frac{dV}{dt} \right)^{-1} \qquad (4)$$

In a typical RC circuit, when charging/discharging the capacitor, the voltage across the capacitor (and additionally the current passing through the capacitor) changes with time according to an exponential relationship (e.g., exponential decay when discharging the capacitor). This can complicate determination of the capacitance of the capacitor as the current and the rate of change of voltage are constantly changing.

Alternatively, the capacitor can be charged/discharged at a constant rate by using a constant current circuit in series with the capacitor. The constant current circuit effectively drives/permits a constant current through the capacitor, such that the rate of change of voltage across the capacitor is also constant. The capacitance of the capacitor can therefore be determined from the division of two measurable constants.

Accordingly, the defibrillator system 100 may comprise a constant current circuit 160 for charging and/or discharging the capacitor 115 at a constant current. The constant current circuit 160 may be formed as part of the defibrillator 120 (as depicted in Fig. 1) or may simply be a part of the defibrillator system 100 and couple to the defibrillator 120 via external connections (e.g., ports) of the defibrillator 120.

The constant current circuit 160 may comprise a constant current source 162 for driving a constant current through the capacitor 115. In particular, the constant current source 162 may function as a power source to charge the capacitor 115 at a constant rate proportional to the applied constant current.

The constant current source 162 may also permit the capacitor 115 to discharge at a constant rate, e.g., by limiting the current flowing through the constant current circuit 160. Additionally, the constant current source may discharge the capacitor 115 at a constant rate by driving a constant current in the direction of discharge. To be more specific, the capacitor 115 may be initially charged (e.g., by the power source 140) to a starting voltage, thereby creating a buildup of charge on one side of the capacitor 115. The constant current source may then drive a constant current that (effectively) transfers the built-up charge from one side of the capacitor 115 to the other. The voltage across the capacitor 115 thus changes at a constant rate due to the constant current.

In some examples, the constant current circuit 160 may comprise an operational amplifier (not visible in Fig. 1) configured to permit the capacitor 115 to discharge at a constant current. For example, by providing a stable reference voltage at the non-inverting input (of the operational amplifier) and connecting a current limiting resistor and a load at the inverting input, a constant current source can be achieved. Note, the operational amplifier may be configured to only maintain the constant current for a predefined time interval, e.g., as the constant current can only be maintained while the capacitor 115 has a sufficient amount of stored charge. Additionally, prior to discharging the capacitor 115, the capacitor may be charged by the power source 140.

The constant current circuit 160 may also comprise a switch 165 that, when closed, permits the capacitor 115 to charge or discharge through the constant current circuit 160. More specifically, when closed, switch 165 forms a closed series circuit comprising the capacitor 115 and the constant current source 162, allowing the capacitor 115 to charge or discharge through the constant current circuit 160 at the constant current.

Preferably, approaches utilizing discharging at a constant current are used to determine the actual capacity of the capacitor 115.

Switch 165 may be controlled by the processing system 110. In other words, the processing system 110 may be configured to open and/or close switch 165, e.g., at predefined times and/or when one or more predefined conditions are met, for the purposes of measuring the actual capacitance of the capacitor 115.

Alternatively, or additionally, switch 165 may be controlled by a user input. For example, the defibrillator 120 may comprise a manual control (not visible in Fig. 1) e.g., a button, which, when acted on by a user, causes switch 165 to open or close, thereby allowing the user to (manually) control charging or discharging of the capacitor 115 through the constant current circuit 160.

In one embodiment, a user acting on the manual control may trigger a mechanical mechanism to open or close switch 165. In another embodiment, a user acting on the manual control may trigger a message/signal to be sent to the processing system 110 that indicates to the processing system 110 to open or close switch 165.

Switches 135, 145 and 165 may be inversely coupled such that only one switch may be closed at a time. In this way, the capacitor 115 may not simultaneously be charged by the power source 140, discharge to provide the energy output to the subject 190, and charge or discharge through the constant current circuit 160. This simplifies operation of the capacitor 115, ensuring the capacitor 115 only charges or discharges through closed series circuits and reduces a risk of damaging components in the circuit.

Note, the above disclosure does not limit switches 135, 145 and 165 from being open at the same time. Particularly, it may be preferable for switches 135, 145 and 165 to be open at the same time when the defibrillator 120 is not in use, e.g., for safety purposes.

The processing system 110 may also be communicatively coupled to the constant current source 162. Specifically, the processing system 110 may be configured to control the constant current source 162 to charge and/or discharge the capacitor 115 at a (predefined) constant current.

The processing system 110 may thus be configured to measure the actual capacitance of the capacitor 115 by performing a process comprising charging or discharging the capacitor 115 at a constant current, i.e., controlling the constant current circuit 160 to charge or discharge the capacitor 115 at the constant current. Subsequently, the processing system 110 may measure (or more specifically calculate) the actual capacitance from the constant current and a measured rate of change of voltage across the capacitor 115.

In this context, calculating the actual capacitance from the constant current and the measured rate of change of voltage across the capacitor 115 will be understood to mean calculating the actual capacitance by processing, by the processing system 110, the constant current and the measured rate of change of voltage across the capacitor 115. This clarification will be understood to apply to other quantities/values/parameters calculated within the present disclosure. In other words, when the phrasing "calculated from" is used, it will be understood to mean "calculated by processing".

The processing system 110 may know the value of the constant current, i.e., the value of the constant current may be predefined, when the processing system is configured to control the constant current source 162. Alternatively, the processing system 110 may measure the constant current using an ammeter (not visible in Fig. 1) in series with the capacitor 115. More specifically, the processing system 100 may be communicatively coupled to the ammeter to receive signals indicative of the value of the constant current.

The processing system 110 may measure the rate of change of voltage across the capacitor 115 by processing voltage measurements received from the voltage measurement system 150, recorded while the capacitor 115 is charging or discharging at the constant current. More specifically, the processing system 110 may control the constant current circuit 160 to charge or discharge the capacitor 115 at the constant current while simultaneously receiving signals from the voltage measurement system 150 indicative of the voltage across the capacitor 115 at different times.

At a minimum, the processing system 110 may measure (or more specifically calculate) the rate of change of voltage across the capacitor 115 from two voltage measurements. As mentioned previously, when charging or discharging a capacitor at a constant current, the voltage across the capacitor changes at a constant rate. In other words, the voltage across the capacitor changes linearly. The rate of change of voltage across the capacitor 115 can thus be calculated from the equation for the gradient of a straight line.

Specifically, when measuring the rate of change of voltage across the capacitor 115, the processing system 110 may receive a first voltage, measured at a first time, and a second voltage, measured at a second time. The rate of change of voltage can thus be calculated from the difference between the first voltage and the second voltage divided by the time interval between the first time and the second time.

As an alternative, when measuring the rate of change of voltage across the capacitor 115, the processing system 110 may receive a plurality of voltages measured at a plurality of respective times. The processing system 100 may then fit a line of best fit to the plurality of voltages plotted against the plurality of respective times (e.g., by a method of least squares) whose gradient is equal to the rate of change of voltage across the capacitor 115. This technique may be preferable when the constant current circuit 160 is not perfect, thus resulting in fluctuations of the constant current, and therefore of the rate of change of voltage, over time. Fitting of the line of best fit effectively averages out these fluctuations, resulting in a more accurate determination of the rate of change of voltage compared to only using two voltage values.

From the above disclosure, the processing system 100 may thus be capable of determining (an estimate of) the actual capacitance of the capacitor 115. The processing system 100 is then configured to determine the initial voltage responsive to the actual capacitance and the target capacitor energy, e.g., from equation (1). The processing system 110 is then further configured to control the defibrillator 120 to charge the capacitor 115 to the initial voltage, e.g., by closing switch 145 until the voltage across the capacitor 115, as measured by the voltage measurement system 150, is equal to the initial voltage.

Appropriate capacitances of the capacitor 115 may be in the range 20 to 150 $\mu$F. Assuming a capacitance equal to 100 $\mu$F, predetermined energies equal to 50 J, 150 J and 200 J may correspond to initial voltages approximately equal to 1152 V, 1995 V and 2304 V, respectively, e.g., when using standard (previously disclosed) values for $R_I$, $R_S$ and $\mu$.

Prior to charging the capacitor 115 to the initial voltage the processing system 110 may be configured to determine whether or not the initial voltage, as determined from the actual capacitance and the target capacitor energy, breaches a threshold voltage. The threshold voltage may, for example, correspond with a maximum rated voltage of the capacitor 115, i.e., a voltage that the manufacturer has indicated that the capacitor 115 should not be charged above. This may be due to safety concerns with charging the capacitor 115 above the threshold voltage, e.g., the capacitor 115 breaking, sparking, and/or becoming hot, potentially resulting in an electrical fire, or that the capacitor 115 begins to leak above the threshold voltage.

Accordingly, in response to the initial voltage breaching the threshold voltage, the processing system 110 may set the initial voltage to be equal to the threshold voltage. For example, after determining the initial voltage from the actual capacitance and target capacitor energy, the processing system 110 may store (a value of) the initial voltage in a memory. Subsequently, in response the initial voltage breaching the threshold voltage, the processing system 110 may overwrite the stored initial voltage with the threshold voltage. The processing system 110 will then charge the capacitor 115 to an initial voltage equal to the threshold voltage, being different to an initial voltage determined from the actual capacitance and target capacitor energy.

After charging the capacitor 115 to the initial voltage, the processing system 110 is configured to control the defibrillator 120 to deliver the predetermined energy to the subject 190 by discharging the capacitor 115. For example, the processing system 110 may close switch 135, causing the capacitor 115 to discharge through the subject 190. The processing system 110 may then open switch 135 once the subject 190 has received the predetermined energy.

The processing system 110 may be configured to open switch 135 in response to one or more predefined conditions being met which are indicative of the subject 190 having received the predetermined energy. For example, the processing system 110 may open switch 135 after a target time has passed since first closing switch 135, said target time being the amount of time required for the subject 190 to receive the predetermined energy. In other words, the processing system 110 may be configured to control the defibrillator to deliver the predetermined energy over a target time.

In some examples, the processing system 110 may be configured to determine the target time responsive to the initial voltage, the final voltage, the actual capacitance, the internal resistance $R_I$, and the predetermined resistance Rs of the subject. In particular, the target time can be calculated from the equation:

$$t = RC \ln\left(\frac{V_0}{V_f}\right) \qquad\qquad (5)$$

where $V_0$ is the initial voltage, $V_f$ is the final voltage, C is the capacitance (e.g., the actual capacitance) and R is the total resistance (e.g., the sum of the internal resistance $R_I$ and the predetermined resistance $R_S$).

The final voltage may be determined responsive to the actual capacitance, the initial voltage, and the total discharge energy. In particular, the final voltage can be calculated from the equation:

$$V_f = \sqrt{V_0^2 - \frac{2W_D}{C}} \qquad\qquad (6)$$

Specifically, equation (6) comes from considering the difference in energy stored by the capacitor 115 between the initial voltage the final voltage (or in other words the energy lost by the capacitor 115 on discharging from the initial voltage to the final voltage).

The processing system 110 may thus time e.g., using a timer or clock (not visible in Fig. 1), for how long switch 135 has been closed, and open switch 135 once the target time has passed, thereby ensuring the subject 190 receives the predetermined energy.

As an alternative, the processing system 110 may instead monitor the voltage across the capacitor 115, e.g., from signals received from the voltage monitoring system 150, as the capacitor 115 discharges through the subject 190. The processing system 110 may then open switch 135 once the voltage across the capacitor 115 reaches the final voltage, corresponding with an expected voltage across the capacitor 115 once the subject 190 has received the predetermined energy.

As part of determining the plurality of disclosed parameters, e.g., the initial voltage, the actual capacitance, the target time, etc., the processing system 110 may be further configured to display (the value(s) of) one or more of said parameters on the user interface 170. In this way, the user is provided information as to the current working state of the defibrillator. For increased contextual understanding, said parameters may also be displayed along with "default" parameters determined responsive to the rated capacitance. The user can therefore more easily tell how much the current parameters have deviated from the default parameters.

In some examples, the processing system 110 may be further configured to print (the value(s) of) one or more of the above-identified parameters via a printer. It is common for defibrillators to comprise a printer for printing information (e.g., ECG information). This printer may be repurposed for communicating the information about derived parameters.

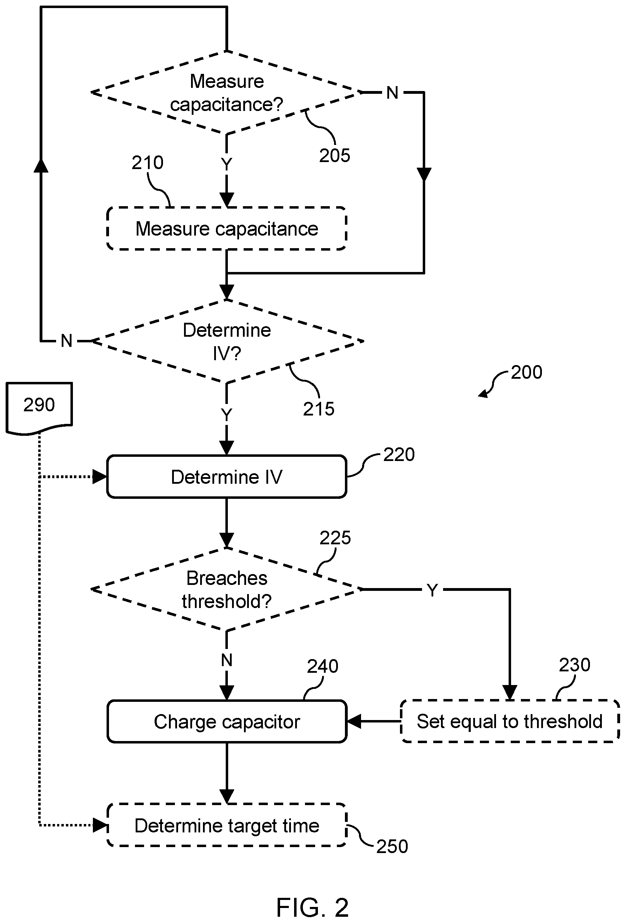

Fig. 2 shows a flowchart illustrating an example method 200 which the processing system 110 of Fig. 1 may be configured to perform. Continued reference will be made to the elements of Fig. 1 to aid in the explanation of how the processing system 110 may perform the method 200. Specifically, the method 200 outlines a process for determining an initial voltage to which to charge the capacitor 115 for delivering the predetermined energy to the subject 190.

The method 200 may comprise a step 210 of measuring the actual capacitance of the capacitor 115. As explained previously, the actual capacitance can be measured by charging or discharging the capacitor 115 at a constant current and measuring the rate of change of voltage across the capacitor 115. Step 210 may therefore comprise charging or discharging the capacitor 115 at a constant current, e.g., by using the constant current circuit 160, while measuring the voltage across the capacitor, e.g., using the voltage measurement system 150, to determine the rate of change of voltage. Step 210 may then further comprise calculating the actual capacitance from the constant current and the rate of change of voltage, e.g., from equation (4).

Prior to step 210, the method 200 may comprise a step 205 of determining whether or not to measure the actual capacitance of the capacitor 115. The determination of step 205 may be subject to one or more criteria. For example, it may be desired that the actual capacitance of the capacitor 115 is measured at least once within a predefined time period, e.g., at least once per hour, at least once per day, at least once per week, etc. Step 205 may therefore have a positive determination (i.e., proceed to step 210) in response to the actual capacitance not being measured within the predefined time period. In other words, the processing system 110 may be configured to measure the actual capacitance (or receive an indication to measure the capacitance) in response to not measuring the actual capacitance within the predefined time period.

Before the predefined time period has passed, the processing system 110 may be in a "standby" state waiting for an indication to measure the actual capacitance. Thus, in some scenarios (e.g. until a predefined time period has elapsed), responsive to a negative determination in step 205, the method may hang at step 205.

It may also be desirable for the actual capacitance of the capacitor 115 to be measured when the defibrillator 120, or the defibrillator system 100 as a whole, is turned on, i.e., when the defibrillator 120 or the defibrillator system 100 receives power. It can be assumed that the defibrillator 120 or defibrillator system 100 will be turned on primarily when a user desires to deliver the predetermined energy output to the subject 190. Therefore, it may be appropriate to measure the actual capacitance at this time to allow an accurate initial voltage to be determined for correctly delivering the predetermined energy to the subject 190.

Accordingly, step 205 may have a positive determination in response to the defibrillator 120 or the defibrillator system 100 being turned on.

In some examples, after measuring the actual capacitance of the capacitor 115, the measured capacitance may be stored in a memory, such that the measured capacitance can be retrieved at a later time, e.g., for the purposes of determining the initial voltage. The stored measured capacitance may be overwritten the next time the actual capacitance of the capacitor 115 is measured, so that the measured capacitance stored by the memory is the most recently measured capacitance.

In line with the above example, step 205 may have a negative determination in response to the defibrillator 120 or the defibrillator system 100 being turned on and the actual capacitance of the capacitor 115 having been recently measured, for example if the actual capacitance had already been measured once within the predefined time period. The method 200 may then, responsive to a negative determination in step 205, skip step 210 and instead retrieve the measured capacitance from the memory. This approach may be appropriate for limiting the number of times the actual capacitance is measured within a given time frame, e.g., as measuring of the capacitance requires charging/discharging of the capacitor 115, which can result in increased/accelerated deterioration of the capacitor 115 if performed too often.

Following step 210, the method 200 may comprise a step 215 of determining whether or not to determine the initial voltage (IV). Step 215 may have a positive determination in response to the defibrillator 120 or the defibrillator system 100 being turned on. Put another way, if the actual capacitance of the capacitor 115 was previously measured (at step 210) in response to the defibrillator 120 or the defibrillator system 100 being turned on, the method 200 may automatically proceed to determining the initial voltage.

Alternatively, or additionally, step 215 may have a positive determination in response to a user input and/or a user provided indicator. For example, the defibrillator 120 may comprise a manual input (e.g., a button) which, when acted on by a user, indicates to the processing system 110 to determine the initial voltage.

In another example, step 215 may have a positive determination in response to a user inputting the predetermined energy into the user interface 170. Furthermore, while the defibrillator 120 or the defibrillator system 100 remains on, but no input/indicator is provided by a user, the processing system 110 may be in a "standby" state waiting for an indication to determine the initial voltage. This is shown in Fig. 2 as a loop from the negative determination of step 215 to the start of step 215.

Step 215 may also have a negative determination in response to the defibrillator 120 or the defibrillator system 100 being turned off, i.e., when the defibrillator 120 or the defibrillator system 100 is not powered or not receiving power. In such a scenario, the method 200 may return to step 205.

The method 200 further comprises a step 220 of determining the initial voltage. More specifically, the initial voltage is determined responsive to the actual capacitance (e.g., from step 210) and the target capacitor energy.

As explained previously, the target capacitor energy may be determined from a lookup table comprising pairs of predetermined energies and corresponding target capacitor energies. Alternatively, the target capacitor energy may be calculated from the predetermined energy, the internal resistance $R_I$, the predetermined resistance Rs and the amplification factor $\mu$, e.g., using equations (2) and (3).

According to the above, step 220 may comprise receiving and/or retrieving one or more parameters 290. More specifically, the processing system 110 may be configured to receive and/or retrieve the one or more parameters 290. The one or more parameters may comprise one or more input parameters, e.g., the predetermined energy, provided via the user interface 170, and/or one or more predefined parameters, e.g., the internal resistance and the predetermined resistance, retrieved from a memory. The initial voltage may then be determined responsive to the one or more input parameters 290 and the actual capacitance.

After step 220, the method 200 may comprise a step 225 of determining whether or not the initial voltage, determined in step 220, breaches the threshold voltage, e.g., if the initial voltage is higher (or lower) than the threshold voltage. Following a positive determination in step 225, the method may proceed to a step 230 of setting the initial voltage to be equal to the threshold voltage.

After step 230, or following a negative determination in step 225, the method 200 proceeds to a step 240 of charging the capacitor 115 to the initial voltage. In particular, following a positive determination in step 225, the capacitor 115 is charged to an initial voltage equal to the threshold voltage. Alternatively, following a negative determination in step 225, the capacitor 115 is charged to an initial voltage equal to the initial voltage determined in step 220.

The method 200 may further comprise, after step 240, a step 250 of determining the target time to discharge the capacitor 115 for when delivering the predetermined energy to the subject 190. As explained previously, the target time may be determined responsive to the initial voltage, the final voltage, the actual capacitance, the internal resistance $R_I$, and the predetermined resistance Rs. Accordingly, step 250 may comprise receiving the one or more input parameters 290 and using the one or more input parameters 290 (in addition to other determined parameters) to determine the target time.

It will be understood that disclosed methods are preferably computer-implemented methods. As such, there is also proposed the concept of a computer program product comprising code means for implementing any described method when said program is run on a processing system connected to a defibrillator having a capacitor. Thus, different portions, lines, or blocks of code of a computer program according to an embodiment may be executed by the processing system to perform herein described methods.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A processing system for a defibrillator configured to deliver a predetermined energy to a subject, wherein an actual capacitance of a capacitor in the defibrillator is different from a rated capacitance of the capacitor and the processing system is configured to:

   determine an initial voltage responsive to the actual capacitance and a target capacitor energy, being a desired energy stored by the capacitor immediately before delivering the predetermined energy to the subject;
   control the defibrillator to charge the capacitor to the initial voltage; and
   control the defibrillator to deliver the predetermined energy to the subject, wherein the predetermined energy is less than the target capacitor energy.

2. The processing system of claim 1, wherein the processing system is configured to:

   measure the actual capacitance; and
   determine the initial voltage responsive to the measured capacitance.

3. The processing system of claim 2, wherein the processing system is configured to measure the actual capacitance by performing a process comprising:

   charging or discharging the capacitor at a constant current; and
   measuring the actual capacitance from the constant current and a rate of change of a voltage across the capacitor.

4. The processing system of any one of claims 1 to 3, wherein the target capacitor energy is associated with the rated capacitance.

5. The processing system of any one of claims 1 to 4, wherein the processing system is configured to, prior to charging the capacitor to the initial voltage:

   determine whether or not the initial voltage breaches a threshold voltage; and
   in response to the initial voltage breaching the threshold voltage, set the initial voltage to be equal to the threshold voltage.

6. The processing system of any one of claims 1 to 5, wherein the processing system is configured to control the defibrillator to deliver the predetermined energy over a target time.

7. The processing system of claim 6, wherein the processing system is configured to determine the target time responsive to the initial voltage, a final voltage, the actual capacitance, an internal resistance of the defibrillator, and a predetermined resistance of the subject, wherein the final voltage is a desired non-zero voltage across the capacitor after delivering the predetermined energy to the subject.

8. The processing system of claim 7, wherein the processing system is configured to determine the final voltage responsive to the actual capacitance, the initial voltage, and a total discharge energy, being an expected energy output by the capacitor when delivering the predetermined energy to the subject.

9. The processing system of claim 8, wherein the total discharge energy is calculated using the formula:

$$W_D = W_S \frac{R_I + R_S}{R_S},$$

wherein $W_D$ is the total discharge energy, $W_S$ is the predetermined energy, $R_I$ is the internal resistance of the defibrillator, and $R_S$ is the predetermined resistance of the subject.

10. The processing system of any one of claims 1 to 9, wherein the processing system is configured to display the initial voltage and the actual capacitance on a user interface communicatively coupled to the processing system.

11. The processing system of any one of claims 1 to 10, wherein the processing system is configured to determine the actual capacitance of the capacitor no less than once per week.

12. A defibrillator system comprising:

the processing system of any one of claims 1 to 10; and
the defibrillator configured to deliver the predetermined energy to the subject.

13. The defibrillator system of claim 12 further comprising:

a power source switchably coupled to the capacitor of the defibrillator for charging the capacitor to an intended voltage;
a voltage measurement system coupled to the capacitor for measuring a voltage across the capacitor; and/or
a constant current circuit switchably coupled to the capacitor, configured to permit the capacitor to charge or discharge at a constant current.

14. A method for charging a capacitor of a defibrillator configured to deliver a predetermined energy to a subject, wherein an actual capacitance of the capacitor in the defibrillator is different from a rated capacitance of the capacitor, wherein the method comprises:

determining an initial voltage responsive to the actual capacitance and a target capacitor energy, being a desired energy stored by the capacitor immediately before delivering the predetermined energy to the subject; and
charging the capacitor to the initial voltage,
wherein the predetermined energy is less than the target capacitor energy.

15. A computer program product comprising computer program code means which, when executed on a processing system connected to a defibrillator having a capacitor, cause the processing system to perform all of the steps of the method according to claim 14.

FIG. 1

FIG. 2

Europäisches
Patentamt
European
Patent Office
Office européen
des brevets

# EUROPEAN SEARCH REPORT

**Application Number**

EP 24 18 3385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 384 544 A (FLUGSTAD BEN [US] ET AL) 24 January 1995 (1995-01-24) | 1,2,4,5, 11-15 | INV. A61N1/39 |
| Y | * abstract; figures 1-4 * <br> * column 1, line 9 - line 57 * <br> * column 2, line 1 - column 3, line 65 * <br> * column 4, line 35 - line 55 * <br> * column 5, line 7 - line 10 * <br> ----- | 3,6-10 | |
| Y | US 5 461 321 A (SANDERS GARY G [US] ET AL) 24 October 1995 (1995-10-24) <br> * abstract * <br> * column 2, line 58 - column 3, line 57 * <br> ----- | 3 | |
| Y | US 2003/125771 A1 (GARRETT MICHAEL C [US]) 3 July 2003 (2003-07-03) <br> * abstract * <br> * paragraphs [0005], [0012] * <br> ----- | 6-9 | |
| Y | US 2021/196965 A1 (TAYLOR TYSON G [US] ET AL) 1 July 2021 (2021-07-01) <br> * abstract; figure 2 * <br> * paragraph [0029] - paragraph [0031] * <br> ----- | 10 | **TECHNICAL FIELDS SEARCHED (IPC)** <br> A61N |

The present search report has been drawn up for all claims

| Place of search | Date of completion of the search | Examiner |
|---|---|---|
| Munich | 5 November 2024 | Minotto, Noël |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 3385

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2024

| Patent document cited in search report | | Publication date | Patent family member(s) | | Publication date |
|---|---|---|---|---|---|
| US 5384544 | A | 24-01-1995 | NONE | | |
| US 5461321 | A | 24-10-1995 | AT | E212726 T1 | 15-02-2002 |
| | | | AU | 677087 B2 | 10-04-1997 |
| | | | CA | 2132225 A1 | 18-03-1995 |
| | | | CN | 1111355 A | 08-11-1995 |
| | | | DE | 69429755 T2 | 26-09-2002 |
| | | | EP | 0644432 A2 | 22-03-1995 |
| | | | ES | 2171426 T3 | 16-09-2002 |
| | | | KR | 950009271 A | 21-04-1995 |
| | | | TW | 247350 B | 11-05-1995 |
| | | | US | 5461321 A | 24-10-1995 |
| | | | US | 5554937 A | 10-09-1996 |
| US 2003125771 | A1 | 03-07-2003 | NONE | | |
| US 2021196965 | A1 | 01-07-2021 | US | 2018339162 A1 | 29-11-2018 |
| | | | US | 2021196965 A1 | 01-07-2021 |

EPO FORM P0459

For more details about this annex : see Official Journal of the European Patent Office, No. 12/82